(19) Europäisches Patentamt
European Patent Office
Office européen des brevets

(11) EP 1 493 425 A1

(12) **DEMANDE DE BREVET EUROPEEN**

(43) Date de publication:
**05.01.2005 Bulletin 2005/01**

(51) Int Cl.[7]: **A61K 7/09**

(21) Numéro de dépôt: **04300404.3**

(22) Date de dépôt: **28.06.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **27.06.2003 FR 0350266**

(71) Demandeur: **L'OREAL**
**75008 Paris (FR)**

(72) Inventeurs:
- **Vic, Gabin**
  **60280 Venette (FR)**
- **Livoreil, Aude**
  **75006 Paris (FR)**
- **Samain, Henri**
  **91570 Bievres (FR)**

(74) Mandataire: **Michelet, Alain et al**
**Cabinet Harlé et Phélip**
**7, rue de Madrid**
**75008 Paris (FR)**

(54) **Composition cosmétique à base de sel(s) de nitrosonium pour la déformation permanente des fibres kératiniques**

(57) L'invention concerne une composition cosmétique pour la déformation permanente des fibres kératiniques, en particulier des cheveux, comprenant, dans un milieu cosmétiquement acceptable, au moins un sel de nitrosonium.

EP 1 493 425 A1

## Description

L'invention se rapporte à une composition cosmétique pour la déformation permanente des fibres kératiniques, en particulier des cheveux.

Généralement, la technique utilisée pour obtenir une déformation permanente des cheveux consiste, dans un premier temps, à réaliser l'ouverture des liaisons disulfures -S-S- de la kératine (cystine) en appliquant sur les cheveux préalablement mis sous tension (bigoudis et autres) une composition réductrice (étape de réduction) puis, de préférence après avoir rincé la chevelure ainsi traitée, à reconstituer dans un second temps lesdites liaisons disulfures en appliquant sur les cheveux toujours sous tension une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est durable dans le temps et résiste notamment à l'action des lavages à l'eau ou par shampooings, et ceci par opposition aux simples techniques classiques de déformation temporaires, telles que les techniques de mise en pli.

Les compositions réductrices utilisables pour la mise en oeuvre de la première étape d'un tel procédé classique de déformation permanente des cheveux (procédé en deux temps) contiennent généralement des composés thiolés, tels que l'acide thioglycolique ou la cystéine, ou des composés générant des ions hydroxydes, tels que la soude ou le carbonate de guanidine par exemple.

Les compositions oxydantes utilisables pour l'étape de fixation sont généralement à base d'eau oxygénée ou de bromates alcalins.

Cependant, une telle technique ne donne pas entièrement satisfaction. En effet, cette technique est très efficace pour modifier la forme des cheveux, mais très dégradante pour les fibres capillaires.

Ainsi, lorsqu'une permanente à l'acide thioglycolique est appliquée sur des cheveux ayant subi une coloration, un très fort décapage de la couleur est observé et la surface des cheveux est fortement dégradée. A l'inverse, si une coloration est appliquée sur des cheveux permanentés, la couleur obtenue est très différente de la couleur normalement obtenue sur des cheveux naturels non permanentés.

Par ailleurs, dans le cas d'un défrisage à la soude, on observe généralement des phénomènes importants de cassure des cheveux.

La présente invention vise donc à résoudre les problèmes ci-dessus, et à fournir une composition cosmétique pour la déformation permanente des cheveux qui réduise fortement la dégradation des cheveux et soit pratiquement sans effet sur une coloration artificielle des fibres kératiniques.

La Demanderesse a trouvé de façon surprenante qu'il était possible de formuler une composition cosmétique remédiant aux inconvénients de l'art antérieur, en utilisant dans un milieu cosmétiquement acceptable au moins un sel de nitrosonium.

La présente invention concerne donc une composition cosmétique pour la déformation permanente des fibres kératiniques, en particulier des cheveux, comprenant, dans un milieu cosmétiquement acceptable, au moins un sel de nitrosonium.

Par déformation permanente, on entend le frisage permanent (encore appelé permanente), le défrisage ou le décrêpage des fi bres kératiniques.

En général, le ou les sels de nitrosonium utilisés dans les compositions selon l'invention sont de formule :

$$n\ NO^{+},\ X^{n-}$$

où n est un entier variant de 1 à 5, et $X^{n-}$ est un anion minéral ou organique, de préférence choisi parmi les anions halogénure, tels que fluorure, bromure, iodure, chlorure, les anions nitrate, nitrite, carbonate, phosphate, phosphonate, sulfate, sulfite, acétate, tétrafluoroborate, perchlorate, $ClO_3^-$, $BrO_3^-$, $FeCl_4^-$, $FeCl_2^-$, $CF_3CO_2^-$, $AlCl_4^-$, $KS_2O_8^-$, $HSO_4^-$, $CF_3SO_3^-$, $CH_3SO_3^-$, $CH_3C_6H_4SO_3^-$, hexachloroantimonate et hexafluorophosphate.

Avantageusement, le ou les sels de nitrosonium représentent de 0,001 à 30 %, de préférence de 0,01 à 20 %, mieux de 0,1 à 10 %, en poids par rapport au poids total de la composition.

Selon un mode de réalisation préféré, la composition selon l'invention comprend en outre au moins un thiol et/ou au moins un disulfure et/ou au moins un sulfite et/ou au moins un bisulfite.

Le ou les thiols utilisables dans la composition selon l'invention peuvent être des monothiols ou des dithiols.

Les monothiols sont de préférence choisis parmi l'acide thioglycolique, les esters de l'acide thioglycolique, l'acide thiolactique, la cystéine, les dérivés de la cystéine, tel que la N-acétylcystéine, la cystéamine et les dérivés de la cystéamine, tel que la N-acétyl cystéamine.

A titre de dithiol, on peut citer la bis mercapto éthylsulfone.

De préférence, les thiols représentent de 0,001 à 30 %, en poids par rapport au poids total de la composition.

Le ou les disulfures utilisables dans la composition selon l'invention sont de préférence de formule :

R-S-S-R'

où R et R' sont identiques ou différents, et représentent

un groupe hydrocarboné, linéaire, ramifié ou cyclique, saturé ou insaturé, pouvant être interrompu par un ou plusieurs hétéroatomes choisis parmi le soufre, l'oxygène, l'azote, le silicium ou le phosphore, et pouvant comprendre un ou plusieurs substituants choisis parmi les groupes hydroxyle, amine, carbamate, carbonate, hydrazine, éther, acide carboxylique, ester, amide, cyano et uréido.

Le ou les disulfures utilisables dans la composition selon l'invention peuvent également être de formule :

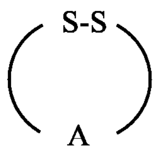

où A est une chaîne hydrocarbonée comprenant 3 à 10 atomes de carbone, linéaire, ramifiée ou cyclique, saturée ou insaturée, pouvant être interrompue par un ou plusieurs hétéroatomes choisis parmi le soufre, l'oxygène, l'azote, le silicium ou le phosphore, et pouvant comprendre un ou plusieurs substituants choisis parmi les groupes hydroxyle, amine, carbamate, carbonate, hydrazine, éther, acide carboxylique, ester, amide, cyano et uréido.

De préférence, le ou les disulfures représentent de 0,001 à 30 %, en poids par rapport au poids total de la composition.

A titre de sulfite utilisable dans la composition selon l'invention, on peut citer le sulfite de sodium. A titre de bisulfite, on peut citer le bisulfite de sodium.

De préférence, le ou les sulfites ou bisulfites représentent de 0,001 à 30 % en poids par rapport au poids total de la composition.

La composition selon l'invention peut également comprendre au moins un chélateur de métaux et/ou au moins un agent réducteur autre que les thiols, dithiols, sulfites et bisulfites décrits ci-dessus.

Par chélateurs de métaux, on entend les composés chimiques ou biologiques ayant la capacité de perturber et de modifier l'environnement ionique en séquestrant les ions métalliques.

Comme autres exemples de chélateurs de métaux utilisables dans la composition selon l'invention, on peut citer :

- l'acide aminotriméthyle phosphonique,
- l'acide β-alanine diacétique,
- l'acide citrique,
- la cyclodextrine,
- l'acide cyclohexanediamine tétracétique,
- l'acide diéthylènetriamine pentaméthylène phosphonique,
- l'acide diéthanolamine N-acétique (diéthanolamine N-acétique),
- l'acide éthylène diamine tétracétique (EDTA ou $YH_4$) et ses sels de sodium ($YH_3Na$, $Y_2H_2Na_2$, $YH_3Na_3$ et $YNa_4$), de potassium ($YH_3K$, $Y_2H_3K_3$ et $YK_4$), de calcium, de disodium, de diammonium et ses sels de triéthanolamine (TEA-EDTA),
- l'acide étidronique,
- l'acide galactanique,
- l'acide hydroxyéthyl éthylènediamine tétracétique (HEDTA) et son sel trisodique,
- l'acide gluconique,
- l'acide glucuronique,
- l'acide nitrilotriacétique (NTA) et son sel trisodique,
- l'acide pentétique,
- l'acide phytique,
- l'acide ribonique,
- le citrate de diammonium,
- l'azacycloheptane diphosphonate de disodium,
- le pyrophoshate de disodium,
- l'hydroxypropyl cyclodextrine,
- le méthyl cyclodextrine,
- le triphosphate de pentapotassium,
- l'aminotriméthylène phosphonate de pentasodium,
- l'éthylènediamine tétraméthylène phosphonate de pentasodium,
- le pentétate de pentasodium,
- le triphosphate de pentasodium,
- le citrate de potassium,
- EDTMP de potassium,
- EDTMP de sodium,
- le chitosane méthylène phosphonate de sodium,
- l'hexamétaphosphate de sodium,
- le métaphosphate de sodium,
- le polyphosphate de potassium,
- le polyphosphate de sodium,
- le trimétaphosphate de sodium,
- le dihydroxyéthylglycinate de sodium,
- le gluconate de potassium,
- le gluconate de sodium,
- le glucopeptate de sodium,
- le glycereth-1 polyphosphate de sodium,
- le pyrophosphate de tétrapotassium,
- le polyphosphate de triéthanolamine (TEA),
- le pyrophosphate de tétrasodium,
- le phosphate de trisodium,
- l'oxyde triphosphonométhylamine de potassium,
- le métasilicate de sodium,
- le phytate de sodium,
- le polydiméthylglycinophénolsulfonate de sodium,
- le tétrahydroxyéthyl éthylène diamine,
- le tétrahydroxypropyl éthylène diamine,
- l'étidronate de tétrapotassium,
- l'étidronate de tétrasodium,
- l'iminodisuccinate de tétrasodium,
- l'éthylènediamine disuccinate de trisodium,
- l'acide éthanolamine N,N-diacétique,
- l'acétate de disodium,
- le dimercaprol,

- la déferoxamine,
- le Zylox, chélateur du fer décrit et revendiqué dans la demande internationale WO 94/61338,
- la néocuproine.

Comme autres exemples de chélateurs de métaux utilisables dans la composition selon l'invention, on peut citer la métallothionéine, la transferrine, la calmoduline, et le chitosane méthylène phosphonate de sodium.

De préférence, le ou les chélateurs de métaux sont choisis parmi l'acide éthylène diamine tétracétique (EDTA) et ses sels de sodium, de potassium, de calcium, de disodium, de diammonium et de triéthanolamine (TEA-EDTA), l'acide hydroxyéthyl éthylène diamine tétracétique (HEDTA) et son sel trisodique, et la néocuproine.

En général, le ou les chélateurs de métaux représentent de 0,00001 à 10 %, en poids par rapport au poids total de la composition.

Comme expliqué précédemment, la composition selon l'invention peut comprendre, outre le ou les chélateurs de métaux, un ou plusieurs agents réducteurs.

Le ou les agents réducteurs utilisables dans la composition selon l'invention, autres que les thiols, dithiols, sulfites et bisulfites décrits précédemment, sont de préférence choisis parmi les ($C_1$-$C_8$) alkyl-phosphines, les réductones dont l'acide ascorbique et l'acide érythorbique.

En général, le ou les agents réducteurs ci-dessus représentent de 0,001 à 30 %, en poids par rapport au poids total de la composition.

Le ou les constituants de la composition selon l'invention doivent être placés dans un milieu cosmétiquement acceptable.

Pour cela, on choisit généralement un ou plusieurs solvants classiquement utilisés dans les compositions cosmétiques. On peut citer les alcools en $C_1$-$C_6$, de préférence les alcanols tels que l'éthanol, le propanol et l'isopropanol, les alcanediols tels que l'éthylèneglycol, le propylène glycol et le pentanediol, l'alcool benzylique, les éthers en $C_1$-$C_6$, les esters en $C_1$-$C_6$, la N-méthylpyrrolidone (NMP) et les cétones en $C_1$-$C_6$.

De plus, avantageusement, le pH de la solution sera compris entre 2 et 13.

Le réglage du pH de la composition peut être obtenu à l'aide d'un agent alcalin, tel que, par exemple, l'ammoniaque, la monoéthanolamine, la diéthanolamine, la triéthanolamine, la 1,3-propanediamine, un carbonate ou bicarbonate alcalin ou d'ammonium, un carbonate organique tel que le carbonate de guanidine, un hydroxyde alcalin, ou bien à l'aide d'un agent acidifiant tel que par exemple l'acide chlorhydrique, l'acide acétique, l'acide lactique, l'acide oxalique ou l'acide borique.

La composition selon l'invention comprend de préférence au moins un adjuvant cosmétiquement acceptable classiquement utilisé dans les compositions cosmétiques destinées à être appliquées sur les fibres kératiniques. On peut citer en particulier les gélifiants et/ou épaississants, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les polymères fixants ou non, les agents bactéricides et les agents anti-pelliculaires.

La composition selon l'invention peut se présenter sous la forme d'une lotion, épaissie ou non, d'une crème, épaissie ou non, d'un gel ou de toute autre forme appropriée. Elle peut éventuellement être conditionnée dans un flacon pompe ou dans un récipient aérosol.

Lorsque la composition selon l'invention est destinée à une opération de défrisage ou de décrêpage des cheveux, la composition se présente de préférence sous forme d'une crème épaissie, par exemple une crème comprenant au moins un composé choisi parmi le stéarate de glycéryle, le stéarate de glycol, une cire auto-émulsionnable ou un alcool gras, ou bien sous la forme d'un liquide ou d'un gel comprenant un ou plusieurs agents épaississants tels que des polymères ou des copolymères carboxyvinyliques qui collent les cheveux et les maintiennent dans la position lisse pendant le temps de pose.

L'invention concerne encore un procédé de déformation permanente des fibres kératiniques, en particulier des cheveux.

Bien que l'exposé qui suit s'articule essentiellement autour du cas particulier du traitement du cheveu, on notera ici que le procédé selon l'invention est applicable à toute fibre kératinique en général, notamment cils, moustaches, poils, et autres.

Dans une première étape, une composition selon l'invention est appliquée sur les fibres kératiniques.

Les cheveux sont mis en forme en utilisant des moyens mécaniques bien connus de l'homme du métier.

Lorsque l'on souhaite réaliser une permanente, on utilise par exemple des bigoudis, la composition selon l'invention étant appliquée avant ou après la pose des bigoudis. De préférence, la composition est appliquée sur des cheveux mouillés préalablement enroulés sur des rouleaux ayant de 4 à 20 mm de diamètre. La composition peut également être appliquée au fur et à mesure de l'enroulage des cheveux.

Lorsque l'on souhaite effectuer le défrisage ou le décrêpage des cheveux, on soumet les cheveux, après application de la composition selon l'invention, à une déformation mécanique permettant de les fixer dans leur nouvelle forme, par une opération de lissage

des cheveux avec un peigne à larges dents, avec le dos d'un peigne ou à la main.

Après l'application de la composition selon l'invention, on laisse poser ladite composition et ladite solution, de préférence pendant 5 à 60 minutes, mieux pendant 5 à 30 minutes, soit à l'air libre, soit à la chaleur, à une température comprise entre 20 et 100°C.

Les cheveux sont alors rincés soigneusement.

La dernière étape du procédé selon l'invention consiste à réaliser la fixation des fibres kératiniques, par oxydation. Cette étape peut se faire soit par mise en contact desdites fibres avec l'oxygène de l'air, soit par application d'une composition comprenant au moins un agent d'oxydation, en général une solution diluée de peroxyde d'hydrogène.

Dans un mode de réalisation préféré du procédé selon l'invention, on applique, après l'application de la composition selon l'invention et avant le temps de pose, une composition comprenant au moins un sel et/ou oxyde métallique, par exemple un sel et/ou oxyde de Cu(I), Cu(II) ou Fe(II).

Comme sels et/ou oxydes de Cu(I), Cu(II) ou Fe(II) utilisables selon l'invention, on peut citer par exemple $Cu_2NO_2$, $Cu_2O$, $CuSO_4$, CuBr, FeO, $FeCl_2$ et $Fe(C_3H_5O_3)_2$.

Dans ce cas, avantageusement, après avoir laissé poser la composition selon l'invention et la solution comprenant au moins un sel et/ou oxyde métallique, on ajoute une solution comprenant au moins un chélateur de métaux. On laisse alors généralement poser, à température ambiante ou sous la chaleur à une température comprise entre 20 et 100°C.

Les étapes de rinçage et de fixation sont alors les mêmes que précédemment.

Le procédé selon l'invention peut être avantageusement mis en oeuvre en utilisant un kit cosmétique, également objet de la présente invention, comprenant une première composition cosmétique telle que définie précédemment et une seconde composition cosmétique comprenant au moins un sel et/ou oxyde métallique, de préférence un sel et/ou oxyde de Cu(I), Cu(II) ou Fe(II), ladite première composition et ladite deuxième composition étant conditionnées séparément dans des compartiments imperméables à l'oxygène.

Selon un mode de réalisation préféré, ledit kit peut en outre comprendre une troisième composition comprenant au moins un chélateur de métaux, ladite troisième composition étant conditionnée séparément dans des compartiments imperméables à l'oxygène.

La présente invention est illustrée par les exemples suivants.

**Exemple 1**

Des mèches de cheveux naturels sont mis en forme en utilisant des bigoudis, puis traités avec une composition cosmétique selon l'invention dont la formulation est la suivante :

- $NOBF_4$ : 0,5 g
- Eau : qsp 100 g

On laisse poser pendant une heure à température ambiante, on rince les cheveux à l'eau, puis on les sèche au casque.

On obtient une mise en forme des cheveux qui résiste aux shampooings.

## Revendications

1. Composition cosmétique pour la déformation permanente des fibres kératiniques, en particulier des cheveux, **caractérisée en ce qu'**elle comprend, dans un milieu cosmétiquement acceptable, au moins un sel de nitrosonium.

2. Composition selon la revendication 1 **caractérisée en ce que** le ou les sels de nitrosonium sont de formule :

$$n\ NO^+,\ X^{n-}$$

où n est un entier variant de 1 à 5, et $X^{n-}$ est un anion minéral ou organique, de préférence choisi parmi les anions halogénure, tels que fluorure, bromure, iodure, chlorure, les anions nitrate, nitrite, carbonate, phosphate, phosphonate, sulfate, sulfite, acétate, tétrafluoroborate, perchlorate, $ClO_3^-$, $BrO_3^-$, $FeCl_4^-$, $FeCl_2^-$, $CF_3CO_2^-$, $AlCl_4^-$, $KS_2O_8^-$, $HSO_4^-$, $CF_3SO_3^-$, $CH_3SO_3^-$, $CH_3C_6H_4SO_3^-$, hexachloroantimonate et hexafluorophosphate.

3. Composition selon la revendication 1 ou 2 **caractérisée en ce que** le ou les sels de nitrosonium représentent de 0,001 à 30 %, de préférence de 0,01 à 20 %, mieux de 0,1 à 10 %, en poids par rapport au poids total de la composition.

4. Composition selon l'une quelconque des revendications 1 à 3 **caractérisée en ce qu'**elle comprend au moins un thiol et/ou au moins un disulfure et/ou au moins un sulfite et/ou au moins un bisulfite.

5. Composition selon 4 **caractérisée en ce que** le ou les thiols sont des monothiols ou des dithiols.

6. Composition selon la revendication 5 **caractérisée en ce que** le ou les monothiols sont choisis parmi l'acide thioglycolique, les esters de l'acide thioglycolique, l'acide thiolactique, la cystéine, les dérivés de la cystéine, la cystéamine et les dérivés de la cystéamine.

7. Composition selon l'une quelconque des revendications 4 à 6 **caractérisée en ce que** le ou les thiols représentent de 0,001 à 30 % en poids par rapport au poids total de la composition.

**8.** Composition selon la revendication 4 **caractérisée en ce que** le ou les disulfures représentent de 0,001 à 30 % en poids par rapport au poids total de la composition.

**9.** Composition selon la revendication 4 **caractérisé en ce que** le ou les sulfites ou bisulfites représentent de 0,001 à 30 % en poids par rapport au poids total de la composition.

**10.** Composition selon l'une quelconque des revendications 1 à 9 **caractérisée en ce qu'**elle comprend au moins un chélateur de métaux.

**11.** Composition selon la revendication 10 **caractérisée en ce que** le
ou les chélateurs de métaux sont choisis parmi l'acide éthylène diamine tétracétique (EDTA) et ses sels de sodium, de potassium, de calcium, de disodium, de diammonium et de triéthanolamine (TEA-EDTA), l'acide hydroxyéthyl éthylène diamine tétracétique (HEDTA) et son sel trisodique, et la néocuproine.

**12.** Composition selon la revendication 10 ou 11 **caractérisée en ce que** le ou les chélateurs de métaux représentent de 0,00001 à 10 % en poids par rapport au poids total de la composition.

**13.** Composition selon l'une quelconque des revendications 1 à 12 **caractérisée en ce qu'**elle comprend au moins un agent réducteur autre que les thiols, dithiols, sulfites, bisulfites définis dans les revendications 4 à 9.

**14.** Composition selon la revendication 13 **caractérisée en ce que** le
ou les agents réducteurs sont choisis parmi les ($C_1$-$C_8$) alkyl-phosphines et les réductones.

**15.** Composition selon la revendication 14 **caractérisé en ce que** les réductones sont choisies parmi l'acide ascorbique et l'acide érythorbique.

**16.** Composition selon l'une quelconque des revendications 13 à 15 **caractérisée en ce que** le ou les agents réducteurs représentent de 0,001 à 30 % en poids par rapport au poids total de la composition.

**17.** Composition selon l'une quelconque des revendications précédentes **caractérisée en ce qu'**elle comprend au moins un solvant choisi parmi les alcools en $C_1$-$C_6$, de préférence les alcanols tels que l'éthanol, le propanol et l'isopropanol, les alcanediols tels que l'éthylèneglycol, le propylène glycol et le pentanediol, l'alcool benzylique, les éthers en $C_1$-$C_6$, les esters en $C_1$-$C_6$, la N-méthylpyrrolidone (NMP), et les cétones en $C_1$-$C_6$.

**18.** Composition selon l'une quelconque des revendications précédentes **caractérisée en ce que** le pH de la composition est compris entre 2 et 13.

**19.** Composition selon l'une quelconque des revendications précédentes **caractérisée en ce qu'**elle comprend un ou plusieurs adjuvants cosmétiques choisis parmi les gélifiants et/ou épaississants, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les polymères fixants ou non, les agents bactéricides, et les agents anti-pelliculaires.

**20.** Procédé de déformation permanente des fibres kératiniques, en particulier des cheveux, comprenant les étapes suivantes :

- appliquer sur les fibres kératiniques une composition telle que définie dans l'une quelconque des revendications 1 à 19 ;
- laisser poser ladite composition et ladite solution, à température ambiante ou à une température comprise entre 20 et 100°C;
- rincer les fibres kératiniques ;
- fixer les fibres kératiniques, soit par mise en contact desdites fibres avec l'oxygène de l'air, soit par application d'une composition comprenant au moins un agent oxydant.

**21.** Procédé selon la revendication 20 **caractérisé en ce que**, après l'application de la composition telle que définie dans l'une quelconque des revendications 1 à 19 et avant de laisser poser, on applique une solution comprenant au moins un sel et/ou oxyde métallique, de préférence un sel et/ou oxyde de Cu(I), Cu(II) ou Fe(II).

**22.** Procédé selon la revendication 21 **caractérisé en ce que** le ou les sels et/ou oxydes de Cu(I), Cu(II) ou Fe(II) sont choisis parmi $Cu_2NO_2$, $Cu_2O$, $CuSO_4$, CuBr, FeO, $FeCl_2$ et $Fe(C_3H_5O_3)_2$.

**23.** Procédé selon la revendication 21 ou 22 **caractérisé en ce que**, après avoir laissé poser et avant

les étapes de rinçage et de fixation, on applique une solution comprenant au moins un chélateur de métaux, et on laisse poser à température ambiante ou à une température comprise entre 20 et 100°C.

**24.** Kit cosmétique pour la déformation permanente des fibres kératiniques, **caractérisé en ce qu'**il comprend une première composition cosmétique telle que définie dans l'une quelconque des revendications 1 à 19 et une seconde composition cosmétique comprenant au moins un sel et/ou oxyde métallique, de préférence un sel et/ou oxyde de Cu (I), Cu(II) ou Fe(II), ladite première composition et ladite deuxième composition étant conditionnées séparément dans des compartiments imperméables à l'oxygène.

**25.** Kit selon la revendication 24 **caractérisé en ce qu'**il comprend une troisième composition comprenant au moins un chélateur de métaux, ladite troisième composition étant conditionnée séparément dans des compartiments imperméables à l'oxygène.

Office européen des brevets

# RAPPORT DE RECHERCHE EUROPEENNE

Numéro de la demande
EP 04 30 0404

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int.Cl.7) |
| X | BUTLER A R ET AL: "NO, nitrosonium ions, nitroxide ions, nitrosothiols and iron-nitrosyls in biology: a chemist's perspective" TRENDS IN PHARMACOLOGICAL SCIENCES, ELSEVIER TRENDS JOURNAL, CAMBRIDGE, GB, vol. 16, no. 1, janvier 1995 (1995-01), pages 18-22, XP004207579 ISSN: 0165-6147 * page 20, colonne GAUCHE * * page 21, colonne DROITE * | 1,2 | A61K7/09 |
| X | DE 11 39 608 B (WELLA AG) 15 novembre 1962 (1962-11-15) * colonne 1, ligne 1-12 * | 1,2 | |
| X | DE 199 45 484 A (KOLB BACHOFEN VICTORIA) 5 avril 2001 (2001-04-05) * exemple 4 * | 1-3 | |
| X | US 5 631 284 A (LEGZDINS PETER ET AL) 20 mai 1997 (1997-05-20) * exemple 5 * | 1-3 | DOMAINES TECHNIQUES RECHERCHES (Int.Cl.7) |
| | | | A61K |
| X | US 6 358 918 B1 (GASSER HARALD ET AL) 19 mars 2002 (2002-03-19) | 1-3 | |
| Y | * colonne 2, ligne 13-34 * * colonne 4, ligne 35-46 * * colonne 5, ligne 51 - colonne 6, ligne 5 * | 4-25 | |
| | -/-- | | |

Le présent rapport a été établi pour toutes les revendications

| Lieu de la recherche | Date d'achèvement de la recherche | Examinateur |
|---|---|---|
| Munich | 29 septembre 2004 | Sala-Jung, N |

CATEGORIE DES DOCUMENTS CITES

X : particulièrement pertinent à lui seul
Y : particulièrement pertinent en combinaison avec un *autre document de la même catégorie*
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : *cité pour d'autres raisons*

& : membre de la même famille, document correspondant

EPO FORM 1503 03.82 (P04C02)

Office européen des brevets

# RAPPORT DE RECHERCHE EUROPEENNE

Numéro de la demande
EP 04 30 0404

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | FR 2 729 852 A (OREAL) 2 août 1996 (1996-08-02) * page 1, ligne 32-37 * * page 3, ligne 16-40 * * page 4, ligne 10-17 * * page 9, ligne 26,37 * * page 9, ligne 41 - page 10, ligne 16 * * page 11, ligne 10-16 * * page 12, ligne 14,15 * ----- | 4-25 | |
| Y | US 5 460 806 A (WHITESIDES GEORGE M ET AL) 24 octobre 1995 (1995-10-24) * colonne 3, ligne 16-38; revendications 4,6,7 * ----- | 4-25 | |
| Y | EP 0 727 202 A (GOLDWELL GMBH) 21 août 1996 (1996-08-21) * page 2, ligne 1,2,32-47,51-59 * * page 3, ligne 1-5,15-20,23-25; revendications 1-7; exemples 1,2 * ----- | 4-25 | DOMAINES TECHNIQUES RECHERCHES (Int.Cl.7) |
| Y | US 6 024 949 A (ROSE BURKHARD) 15 février 2000 (2000-02-15) * colonne 1, ligne 1-3,53-59 * * colonne 2, ligne 9-22; revendications 3,5; exemples 1-7 * * colonne 3, ligne 3-10 * ----- -/-- | 4-25 | |

Le présent rapport a été établi pour toutes les revendications

| Lieu de la recherche | Date d'achèvement de la recherche | Examinateur |
|---|---|---|
| Munich | 29 septembre 2004 | Sala-Jung, N |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 04 30 0404

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | ARNELLE D R ET AL: "NO+, NO., AND NO- DONATION BY S-NITROSOTHIOLS: IMPLICATIONS FOR REGULATION OF PHYSIOLOGICAL FUNCTIONS BY S-NITROSYLATION AND ACCELERATION OF DISULFIDE FORMATION" ARCHIVES OF BIOCHEMISTRY AND BIOPHYSICS, NEW YORK, US, US, vol. 318, no. 2, 20 avril 1995 (1995-04-20), pages 279-285, XP000607903 ISSN: 0003-9861 * page 284, colonne GAUCHE, ligne 17-23 * ----- | | |

DOMAINES TECHNIQUES RECHERCHES (Int.Cl.7)

Le présent rapport a été établi pour toutes les revendications

| Lieu de la recherche | Date d'achèvement de la recherche | Examinateur |
|---|---|---|
| Munich | 29 septembre 2004 | Sala-Jung, N |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 04 30 0404

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-09-2004

| Document brevet cité au rapport de recherche | | Date de publication | Membre(s) de la famille de brevet(s) | | | Date de publication |
|---|---|---|---|---|---|---|
| DE 1139608 | B | 15-11-1962 | AUCUN | | | |
| DE 19945484 | A | 05-04-2001 | DE | 19945484 | A1 | 05-04-2001 |
| | | | AU | 6997200 | A | 24-04-2001 |
| | | | WO | 0121148 | A1 | 29-03-2001 |
| | | | EP | 1216019 | A1 | 26-06-2002 |
| US 5631284 | A | 20-05-1997 | WO | 9829115 | A1 | 09-07-1998 |
| | | | US | 5811463 | A | 22-09-1998 |
| | | | AU | 1135597 | A | 31-07-1998 |
| US 6358918 | B1 | 19-03-2002 | AT | 405135 | B | 25-05-1999 |
| | | | AT | 6897 | A | 15-10-1998 |
| | | | AT | 262917 | T | 15-04-2004 |
| | | | DE | 59811071 | D1 | 06-05-2004 |
| | | | EP | 0853944 | A2 | 22-07-1998 |
| | | | US | 6124255 | A | 26-09-2000 |
| FR 2729852 | A | 02-08-1996 | FR | 2729852 | A1 | 02-08-1996 |
| | | | AT | 163538 | T | 15-03-1998 |
| | | | AU | 674640 | B2 | 02-01-1997 |
| | | | AU | 4201496 | A | 08-08-1996 |
| | | | BR | 9600510 | A | 30-12-1997 |
| | | | CA | 2168323 | A1 | 31-07-1996 |
| | | | CN | 1138450 | A | 25-12-1996 |
| | | | DE | 69600174 | D1 | 09-04-1998 |
| | | | DE | 69600174 | T2 | 10-06-1998 |
| | | | DK | 723772 | T3 | 21-12-1998 |
| | | | EP | 0723772 | A1 | 31-07-1996 |
| | | | ES | 2116132 | T3 | 01-07-1998 |
| | | | GR | 3026421 | T3 | 30-06-1998 |
| | | | HU | 9600193 | A2 | 28-04-1997 |
| | | | JP | 2960344 | B2 | 06-10-1999 |
| | | | JP | 8231355 | A | 10-09-1996 |
| | | | KR | 190936 | B1 | 01-06-1999 |
| | | | PL | 312526 | A1 | 05-08-1996 |
| | | | RU | 2135157 | C1 | 27-08-1999 |
| | | | US | 5932201 | A | 03-08-1999 |
| US 5460806 | A | 24-10-1995 | AUCUN | | | |
| EP 0727202 | A | 21-08-1996 | DE | 4428575 | C1 | 11-04-1996 |
| | | | AT | 143798 | T | 15-10-1996 |
| | | | DE | 59500028 | D1 | 14-11-1996 |
| | | | EP | 0727202 | A1 | 21-08-1996 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 04 30 0404

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-09-2004

| Document brevet cité au rapport de recherche | Date de publication | Membre(s) de la famille de brevet(s) | Date de publication |
|---|---|---|---|
| US 6024949 A | 15-02-2000 | AT 98471 T | 15-01-1994 |
| | | DE 59200033 D1 | 27-01-1994 |
| | | DK 515768 T3 | 18-04-1994 |
| | | EP 0515768 A2 | 02-12-1992 |
| | | ES 2048599 T3 | 16-03-1994 |

EPO FORM P0460

Pour tout renseignement concernant cette annexe : voir Journal Officiel de l'Office européen des brevets, No.12/82